# EUROPEAN PATENT APPLICATION

(11) **EP 3 943 130 A1**
(43) Date of publication of application: **26.01.2022**
(21) Application number: 19919593.4
(22) Date of filing: 30.09.2019
(51) Int. Cl.: A61M 1/00

(54) **NEGATIVE PRESSURE DRAINAGE AND CLEANING SYSTEM FOR SUTURELESS CLOSURE OF SKIN WOUND**

(30) Priority: 19.03.2019 CN 201910208814; 17.05.2019 CN 201910414748
(71) Applicant: Jingrun (Shanghai) Medical Instruments Co., Ltd., Pudong New District, Shanghai 201306 (CN)
(72) Inventor: CHEN, Wei, Pudong New District Shanghai 201306 (CN)
(74) Representative: Herzog, Markus
(86) International application number: PCT/CN2019/109565
(87) International publication number: WO 2020/186729

(57) **Abstract**

A negative pressure drainage and cleaning system for sutureless closure of a skin wound. The system comprises: a flushing device (1), which comprises a conveying pump (103) and a first catheter (105) partially probing into a subcutaneous wound internal cavity (8) at a preset depth and is used for conveying a cleaning solution by means of the first catheter (105) to the preset depth of the subcutaneous wound internal cavity (8); a negative pressure device (2), which comprises a negative pressure source (202) and a second catheter (205) partially probing into the subcutaneous wound internal cavity (8) at a preset depth and is used for generating negative pressure to force the subcutaneous wound internal cavity (8) to be closed during healing and draining effusion in the subcutaneous wound internal cavity (8) by means of the second catheter (205); a control device (3), electrically connected to the flushing device (1) and the negative pressure device (2) and used for maintaining a vacuum degree of the subcutaneous wound internal cavity (8) by controlling the output pressure or flow of the flushing device (1) and the negative pressure device (2) according to the received feedback. The system can assist to implement the sutureless closure of a full-thickness tissue above the deep fascia of the skin, avoids horizontal scars on the skin surface caused by suture compression/cutting, and leaves no suture knots in the superficial fascia, thereby eliminating an important factor which causes colonization of bacteria and a main cause of recurrence of wound infection.

## Description

### TECHNICAL FIELD

The present application relates to the field of medical apparatus and instruments, particularly to a negative pressure drainage and cleaning system for sutureless closed skin incisions.

### BACKGROUD ART

As we all know, humans have gradually lost an ability to regenerate a body part in the process of evolution. Scar healing is the main way to deal with tissue damage caused by trauma. Therefore, there is a close relationship between surgical suturing and tissue healing. To a certain extent, the quality of suturing can determine the quality of tissue healing. It is common to use a curved needle carrying sutures pass through the transecting tissues in surgical suturing, and then tighten and fix the sutures by knotting, so that the transecting tissues fit closely, thereby creating favorable conditions for healing. The ideal surgical suturing should meet the requirements of moderate tension, good incision edge alignment, no dead space, no permanent or only a few sutured marks. According to the structure and physiological characteristics of different tissues, the requirements for surgical suture are different. The selection of suturing materials and manners greatly affects the surgical effect. When the tissue is healed and its functional strength is restored, the suturing material loses its functional significance. At this moment, the sutures on the body surface should be removed. Large numbers of clinical medicine prove that it is difficult to remove the sutures left in the deep tissues caused by suturing after the incision is healed. The result is that the sutures will be wrapped by scar tissues, or being degraded and absorbed within a certain period of time. These knots left in the deep tissues sometimes affect the human body. In addition, the significant scars caused by traditional surgical suturing also have a great impact on aesthetics.

Currently, there are the following ways to avoid the suture scars on the surface of the skin: 1, intradermal suture method: after completing the suturing of superficial fascia, a surgeon uses sutures to make continuous intradermal suturing in the superficial layer of dermis, and tighten the sutures to align the edge of skin; 2, tissue glue method: also after the superficial fascia suturing is completed, the edge of the skin is adhered with methyl acrylate tissue glue; 3, skin tension reducer (commonly known as a skin zipper): after the superficial fascia suturing is completed, a tension reducing tape with a locking structure is used to pull the skin tightly and tighten the edges of the skin. However, as to the current types of solutions, a large number of suturing in the superficial fascia layer of the skin is still needed, which cannot avoid a series of safety problems caused by residual sutures. Safe and aesthetically pleasing closed skin incisions have become a technical problem that those skilled in the art are expected to solve.

### SUMMARY OF THE INVENTION

In view of the above-mentioned shortcomings in the prior art, the object of the present application is to provide a negative pressure drainage and cleaning system for sutureless closed skin incisions, which is used for solving the problems in the prior art that the suturing of incisions easily leaves a trace after operation and effusions are difficult to discharge.

In one aspect, the negative pressure drainage and cleaning system for sutureless closed skin incisions, comprising: an irrigation device, comprising a delivery pump and a first catheter partially reaching into a preset depth of inner cavity of a subcutaneous incision, and used for delivering irrigation solution to the preset depth of the inner cavity of the subcutaneous incision through the first catheter; a negative pressure device, comprising a negative pressure source and a second catheter partially reaching into a preset depth of the inner cavity of the subcutaneous incision, and used for generating a negative pressure to force the inner cavity of the subcutaneous incision to be in a closed state during the healing process and to drain effusions in the inner cavity of the subcutaneous incision through the second catheter; and a control device, electrically connected with the irrigation device and the negative pressure device, and used for controlling output pressure or flow of the irrigation device according to received feedback and the negative pressure device, so as to maintain vacuum degree of the inner cavity of the subcutaneous incision.

In some embodiments, the irrigation device comprises: an irrigation solution container, storing the irrigation solution for irrigating the inner cavity of the subcutaneous incision, and communicating with the delivery pump through the first catheter; a first pressure sensor, used for feeding back sensed fluid pressure value of the first catheter between the delivery pump and the inner cavity of the subcutaneous incision to the control device, so as to adjust rotation speed of the delivery pump to control the output pressure of the irrigation solution; and a first vacuum sensor, used for sensing vacuum degree of the first catheter between the delivery pump and the irrigation solution container, so as to monitor exhausting state of the irrigation solution in the irrigation solution container.

In some embodiments, the irrigation device comprises an alarming device used for outputting an alarming signal when the first vacuum sensor monitors that the irrigation solution in the irrigation solution container is exhausted.

In some embodiments, the delivery pump is a diaphragm pump.

In some embodiments, the negative pressure device comprises: a collection container, used for collecting the effusions drained by the second catheter from the inner cavity of the subcutaneous incision; a second pressure sensor, used for sensing fluid resistance of the second catheter between the negative pressure source and the collection container, so as to monitor filling state of the collection container; a second vacuum sensor, used for feeding back the sensed vacuum degree of the second catheter between the negative pressure source and the inner cavity of the subcutaneous incision to the control device, so as to adjust power of the negative pressure source to control generated negative pressure.

In some embodiments, the negative pressure source is a diaphragm pump.

In some embodiments, the preset depth is an interval depth from superficial fascia to a fat layer and a deep fascia layer in tissues of skin.

In some embodiments, the first or second catheter partially reaches into the preset depth of the inner cavity of the subcutaneous incision via a preset position of a skin incision; or the first or second catheter passes through skins and subcutaneous tissues via a preset position far away from a skin incision to partially reach into the preset depth of the inner cavity of the subcutaneous incision.

In some embodiments, the first catheter and the second catheter are integrated, or the second catheter is sleeved in the first catheter.

In some embodiments, the wall of the part of the first or second catheter reaching into the preset depth in the skin incision is provided with a plurality of through holes.

In some embodiments, the first or second catheter is provided with a check valve.

In some embodiments, the check valve is a duckbill valve or a quadrant valve.

In some embodiments, the negative pressure device is also used for maintaining tissue positions on surface and peripheral areas of the skin incision to be stable by virtue of the negative pressure that generated.

In some embodiments, the negative pressure device comprises: a sealing membrane, used for adhering to skins and covering surface of the skin incision to form a sealed space; and a negative pressure channel, communicating the negative pressure source, wherein the sealing membrane provides a shrinkage force to drive opposite shrinkage of the skin incision through the negative pressure generated by the negative pressure source.

In some embodiments, the sealing membrane is adhered to the surface of the skin through adhesion layer of the sealing membrane relative to the surface of the skin, and material of the adhesion layer comprises a flexible impermeable material of polyurethane coated with an acrylic adhesive.

In some embodiments, the sealing membrane is provided with an observation window made of a light transmitting material.

In some embodiments, the negative pressure device comprises a humidity detection component used for providing detected humidity information in the sealed space to the negative pressure source so as to facilitate adjustment of the negative pressure output of the negative pressure source.

In some embodiments, the second catheter comprises subcutaneous drainage holes and negative pressure holes above skin, the subcutaneous drainage holes are a plurality of through holes formed on the wall of the part of the second catheter reaching into the preset depth in the skin incision; and the negative pressure holes above skin communicate with the sealed space to apply a negative pressure to the sealed space.

Compared with the prior art, the present application has the beneficial effects that by using the negative pressure drainage and cleaning system for sutureless closed skin incisions in the present application, in a first aspect, by means of a negative pressure drainage device, the inner cavity of a subcutaneous incision is forced to be kept in a closed state during healing and rehabilitation process, transecting tissues are maintained in a fitting state, and oozed blood and oozed fluid in the incision cavity are timely cleared by continuous negative pressure suction; on this basis, a liquid drug is intermittently delivered to the inner cavity of the subcutaneous incision via the cleaning system, so that solidified blood clots in the inner cavity of the subcutaneous incision are moisturized to facilitate removal and the potential bacterial community reaching a colonization concentration is diluted and cleared along with irrigation solution in drainage, so as to maintain the clean condition in the incision cavity; in a second aspect, a negative pressure is generated through negative pressure drainage to maintain tissue position in the skin incision and its peripheral areas to be stable so as to facilitate tissue healing. In addition, under the assist of the present application, sutureless closure of full-thickness tissues above deep fascia of skin can be achieved, so as to avoid transverse scars (commonly called "centipede foot") caused by suture oppression and cutting on the surface of the skin, and no suture knots are left in superficial fascia, so as to eliminate an important factor causing bacterial colonization and a main inducement leading to relapse of incision infection.

### BRIEF DESCRIPTION OF THE DRAWINGS

Fig. 1 is a schematic diagram of an embodiment of a negative pressure drainage and cleaning system for sutureless closed skin incisions of the present application.
Fig. 2 is a schematic diagram of an embodiment of a negative pressure drainage and cleaning system for sutureless closed skin incisions of the present application when in application.
Fig. 3 is a schematic diagram of an embodiment of an alarming device in a negative pressure drainage and cleaning system for sutureless closed skin incisions of the present application.
Fig. 4 is a schematic diagram of an embodiment in which a first or second catheter partially reaches into a preset depth of the inner cavity of the subcutaneous incision from a preset position of a skin incision in the present application.
Fig. 5 is a schematic diagram of an embodiment in which a first or second catheter passes through skins and subcutaneous tissues via a preset position far away from a skin incision in the present application.
Fig. 6 is a schematic diagram of an embodiment of an irrigation device in the present application.
Fig. 7 is a schematic diagram of an embodiment of a negative pressure device in the present application.
Fig. 8 is a structural schematic diagram of a closure member in an incision closing device in an embodiment of the present application.
Fig. 9 is an embodiment schematic diagram of combination of a closure member and an incision in an embodiment of the present application.
Fig. 10 is a schematic diagram of another embodiment of a closure member of an incision closing device in the present application.
Fig. 11 is a schematic diagram of application of a closure member and an auxiliary member of an incision closing device in an embodiment of the present application.
Fig. 12 is a structural schematic diagram of a closure member of an incision closing device in another embodiment of the present application.
Fig. 13 is a structural schematic diagram of an embodiment of first and second catheters of the present application.
Fig. 14 is a structural schematic diagram of another embodiment of first and second catheters of the present application.
Fig. 15 is a schematic diagram of a sealing and stress structure of a negative pressure device in an embodiment of the present application.
Fig. 16 is a schematic diagram of an embodiment of a second catheter in the present application.
Fig. 17 is a schematic diagram of an application embodiment of the present application.

### DETAILED DESCRIPTION OF THE EMBODIMENTS

Implementations of the present application will be described below through specific embodiments, and those skilled in the art can easily understand other advantages and effects of the present application from the contents disclosed in the present specification.

In the following description, several embodiments of the present application are described with reference to the attached figures. It should be understood that other embodiments may also be used, and changes of mechanical composition, structure, electric and operation may be made without departing from the spirit and scope of this application. The following detailed description should not be considered restrictive, and the scope of the implementation of this application is limited only by the published patent claims. The terminology used herein is intended only to describe specific embodiments and is not intended to restrict this application. Spatial terms, such as "up", "down", "left", "right", "lower part", "higher part", "bottom", "below", "above", "upwards", etc., can be used in this application to illustrate the relationship between one element and another or one feature and another shown in the figure.

Although in some embodiments the terms first and second are used in this article to describe various elements or parameters, these elements or parameters should not be restricted by these terms. These terms are used only to distinguish one element or parameter from another element or parameter. For example, the first catheter may be referred to as the second catheter, and similarly, the second catheter may be referred to as the first catheter without leaving the scope of the various described embodiments. Both the first catheter and the second catheter describe a catheter, but they are not the same catheter unless the context otherwise makes it clear.

Furthermore, as used herein, such single forms as "one", "a" and "the" aim at also including the plural forms, unless contrarily indicated in the text. It should be further understood that, such terms as "comprise" and "include" indicate the existence of the features, steps, operations, elements, components, items, types and/or groups, but do not exclude the existence, emergence or addition of one or more other features, steps, operations, elements, components, items, types and/or groups. The terms "or" and "and/or" used herein are explained to be inclusive, or indicate any one or any combination. Therefore, "A, B or C" or "A, B and/or C" indicates "any of the following: A; B; C; A and B; A and C; B and C; A, B and C". Exceptions of the definition only exist when the combinations of elements, functions, steps or operations are mutually exclusive inherently in some ways.

Surgeons used to divide the skin into a superficial fascia layer and a deep fascia layer. The superficial fascia refers to skin epidermis, dermis and subcutaneous fat tissues. Specifically, the superficial fascia is formed by continuation of the superficial fascia in the anterior and lateral regions of the chest and the superficial fascia of the neck, abdomen and upper limbs, and contains fat, superficial blood vessels, lymphatic vessels, cutaneous nerves and mammary glands. The deep fascia refers to fibrous connective tissues that separate the skin from muscle tissues. During the surgery, in the skin suturing stage, the superficial fascia and the deep fascia are usually sutured layer by layer in an order that the deep fascia is sutured first, then the subcutaneous tissue is sutured, and finally the skin is sutured. The significance of layered suturing is to resist against skin tension layer by layer in order to improve the quality of healing. Since skin suture can cause ischemia of local tissues, scars, commonly known as "centipede foot", may be formed in the direction perpendicular to the incision at the suture site after surgery, which is an inevitable result brought by the traditional suturing method. In addition, the conventional skin suturing also necessarily leave a large number of knots in the subcutaneous superficial fascia layer, which often leads to incision complications due to the existence of knots, such as incision infection, fat liquefaction, etc., thereby causing serious consequences and affecting the quality of life of sufferers.

As to the treatment of incisions (also known as incisions or surgical incisions, wounds, etc.) inevitably caused during surgeries of patients (sufferers), suturing is often used to help healing of the incisions of patients. In the treatment process, that is, after the incision is sutured with needles and threads, in order to facilitate healing of the incision, the decompression dressing components are applied in the closure force in the industry, the decompression dressing component includes a pillow body formed by a closed pillow material and having a closing member, and the closing member can generate an inward closing force when the closed dressing pillow is placed under decompression. In some cases, the component also includes a wicking material having a fluid flow path for removing fluid. However, this method still needs to suture the incision in the application process, which can only play a role in helping suturing and healing, and cannot completely replace the suturing step, so it will still leave suturing traces on the skin of the patient. In addition, the closing force produced in the structure can only act on the surface layer. When the incision is deep, the deep subcutaneous muscle tissue cannot be stressed to be in a closed state, and the wicking material can only deal with the fluid oozed from the surface of the skin, and cannot deal with subcutaneous effusions (for example, oozed blood and oozed fluid) in time, thereby being not conducive to the recovery of the incision.

In view of this, the present application provides a negative pressure drainage and cleaning system for sutureless closed skin incisions, in order to achieve the control of environments in and around skin incisions to facilitate healing of the skin incisions. In the embodiments provided below, a negative pressure drainage and cleaning system for sutureless closed skin incisions of the present application comprises: an irrigation device, a negative pressure device, and a control device. The irrigation device comprises a delivery pump and a first catheter, wherein the first catheter partially reaches into a preset depth of the inner cavity of the subcutaneous incision, and the delivery pump delivers irrigation solution to the first catheter, so as to deliver the irrigation solution to the inner cavity of the subcutaneous incision while cleaning the inner cavity of the subcutaneous incision to promote the healing of the inner cavity of the subcutaneous incision. The negative pressure device comprises a negative pressure source and a second catheter, wherein the second catheter partially reaches into a preset depth of the inner cavity of the subcutaneous incision, and the negative pressure source communicates with the second catheter to generate a negative pressure to force the inner cavity of the subcutaneous incision to be in a closed state during the healing process, and meanwhile drain the effusions in the inner cavity of the subcutaneous incision to ensure that the environment in the inner cavity of the subcutaneous incision is healthy. The control device is electrically connected with the irrigation device and the negative pressure device, thereby controlling the irrigation device and the negative pressure device. Meanwhile, the irrigation device and the negative pressure device are adjusted through the information fed back from the irrigation device and the negative pressure device, thereby adjusting the output pressure or flow of the irrigation device and the negative pressure device.

In the present application, by means of a negative pressure drainage and cleaning system for sutureless closed skin incisions, in one aspect, the inner cavity of the subcutaneous incision is kept in a closed state during the healing and rehabilitation process through a negative pressure device, transecting tissues are maintained in a fitting state, and oozed blood and oozed fluid in the incision cavity are timely cleared by continuous negative pressure suction. Meanwhile, in the present application, tissue position in the skin incision and its peripheral areas is maintained stable through the negative pressure generated by the negative pressure device, so as to facilitate the healing of tissues. In a second aspect of the present application, a liquid drug is intermittently delivered to the inner cavity of the subcutaneous incision via the irrigation device, so that solidified blood clots in the inner cavity of the subcutaneous incision are moisturized to facilitate removal and the potential bacterial community reaching a colonization concentration is diluted and cleared along with irrigation solution in drainage, so as to maintain the clean condition in the incision cavity. In a third aspect of the present application, the irrigation device and the negative pressure device are controlled through the feedback mechanism of the control device to guarantee the irrigation intensity, flow and negative pressure intensity, to ensure causing no adverse influence on the patient and to maintain the vacuum degree of the inner cavity of the subcutaneous incision. In addition, in the present application, the inner cavity of the subcutaneous incision is closed to assist in achieving the sutureless closure of full-thickness tissues above deep fascia of the skin, so as to avoid transverse scars on the surface of the skin caused by suture oppression and cutting, and no suture knots are left in superficial fascia, so as to eliminate an important factor causing bacterial colonization and a main inducement leading to relapse of incision infection.

It should be understood that, in embodiments concerned in the present application, the skin incision comprises cracks formed by continuous interruption of all the skins or other tissue parts, and widely refers to incisions, wounds, damages or other therapeutic targets located on or in tissues. It should be noted that in most cases, the skin incision is caused by surgery. However, in some cases, the skin incision may also be caused by accidents such as cuts or collisions.

It should be understood that the healing of skin incisions needs to fit the two sections of the inner cavity of the subcutaneous incision, for example, it can be achieved by suturing and other means in the prior art. In the present embodiment, gas in the inner cavity of the subcutaneous incision is extracted by the negative pressure to maintain a certain vacuum degree in the inner cavity of the subcutaneous incision, so that the two sections of the inner cavity of the subcutaneous incision are fit inwardly, thereby realizing the closure of the skin incision.

In some embodiments, the tissues comprise but are not limited to bone tissues, fat tissues, muscle tissues, nerve tissues, skin tissues, vascular tissues, connective tissues, cartilages, tendons or ligaments. The incision can comprise, for example, chronic, acute, traumatic, subacute and dehiscent incisions; partial cortical burns and ulcers (such as diabetic ulcers, pressure ulcers or venous insufficiency ulcers), flaps, and grafts. The term "tissue part" can also refer to any tissue area that is not necessarily injured or damaged, but is an area in which it may wish to increase or promote the growth of other tissues. For example, a negative pressure can be applied to the tissue part so as to grow additional tissues that can be obtained and transplanted.

It should be understood that sutureless in the present application refers to a treatment means in which no needles and no suturing threads are used to suture the superficial fascia of the skin in the treatment of closing skin incision of the superficial fascia of the skin (i.e., skin epidermis, dermis and subcutaneous fat tissue parts) of the skin or the above superficial fascia layer of the skin, or in the healing process of the skin incision, or in other treatments of the skin incisions such as washing, disinfecting and topical application of drug after surgeries. In these processes, medical tools such as suturing needles and suturing threads are not used, therefore after the skin incision is healed, there is no process of clearing foreign matters in the incision or the incision surface of the superficial fascia of the skin, for example, a step and operating process of dismantling or removing the sutures or taking out the suture residues.

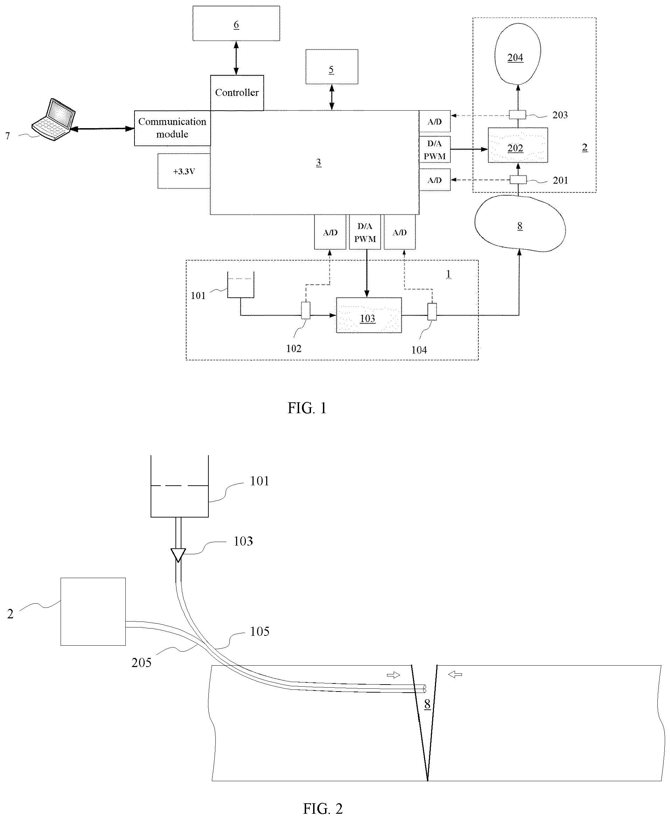

In an exemplary embodiment, please refer to Fig 1. Fig. 1 is a schematic diagram of an embodiment of a negative pressure drainage and cleaning system for sutureless closed skin incisions of the present application. As shown in the figure, the negative pressure drainage and cleaning system for sutureless closed skin incisions of the present application comprises an irrigation device 1, a negative pressure device 2 and a control device 3.

In an exemplary embodiment, please refer to Fig. 2 which is a schematic diagram of an embodiment of a negative pressure drainage and cleaning system for sutureless closed skin incisions of the present application when in application. As shown in the figure, the irrigation device 1 comprises a delivery pump 103 and a first catheter 105 partially reaching into a preset depth of the inner cavity 8 of the subcutaneous incision, and is used for delivering irrigation solution to a preset depth of the inner cavity 8 of the subcutaneous incision through the first catheter 105.

It should be understood that the inner cavity 8 of the subcutaneous incision refers to a cavity formed by the internal incision under the incision during the operation. The cavity comprises all the gaps formed by the continuity interruptions of skin or other tissue sites. Because the organism tissues of human body or animal body have elastic or flexible performance, in the actual state, the inner cavity 8 of the subcutaneous incision, namely the gap, is not necessarily directly present as being in a cavity state or hollow state, therefore, the shape and size of the gap are not limited in the embodiment provided in the present application.

It should be understood that the "catheter" disclosed in the present application refers to a fact that components can be in fluid linkage with each other so as to provide a path for fluid (i.e., liquid and/or gas) transfer among these components. For example, these components can be in fluid linkage through a fluid conducting device (such as a pipe). The "catheter" used herein widely comprises a pipe, a pipeline, a hose, a conduit or other structures having one or more pipe cavities adaptive to deliver fluid between two terminals. Typically, the pipe has some flexible, long and narrow cylindrical structures, but the geometrical shape and rigidity can be changed. In some embodiments, many components can be linked by virtue of physical access to form a single structure as a whole, or are made of the same material. In addition, some fluid conducting devices can be modulated to other components or combined with other components in other manners.

The preset depth is an interval depth from the superficial fascia to the fat layer and the deep fascia layer in the skin tissue. Wherein, in a preferred embodiment, the preset depth position is at the junction between the fat layer and the deep fascia layer in the skin tissue. It should be understood that a blood vessel network, a superficial fascia layer, fat, a deep fascia layer, muscles and bones are located under the skin tissue in sequence. Therefore, the preset depth in the present embodiment comprises the depth interval from the superficial fascia layer to the fat and the deep fascia layer.

In an exemplary embodiment, the irrigation solution comprises fluids that can be used to clean the incisions, such as distilled water, hydrogen peroxide and physiological saline. In some embodiments, the irrigation solution may also comprise a liquid medicine which is determined according to different causes and different types of inner cavities 8 of subcutaneous incisions. For example, when bacterial infection occurs in the incision, antibiotics are added into the irrigation solution; when inflammation occurs in the incision, liquid medicines having antiinflammatory ingredients can be added into the irrigation solution. In some other embodiments, the solid medicines can also be dissolved into the irrigation solution to be delivered to the inner cavity 8 of the subcutaneous incision.

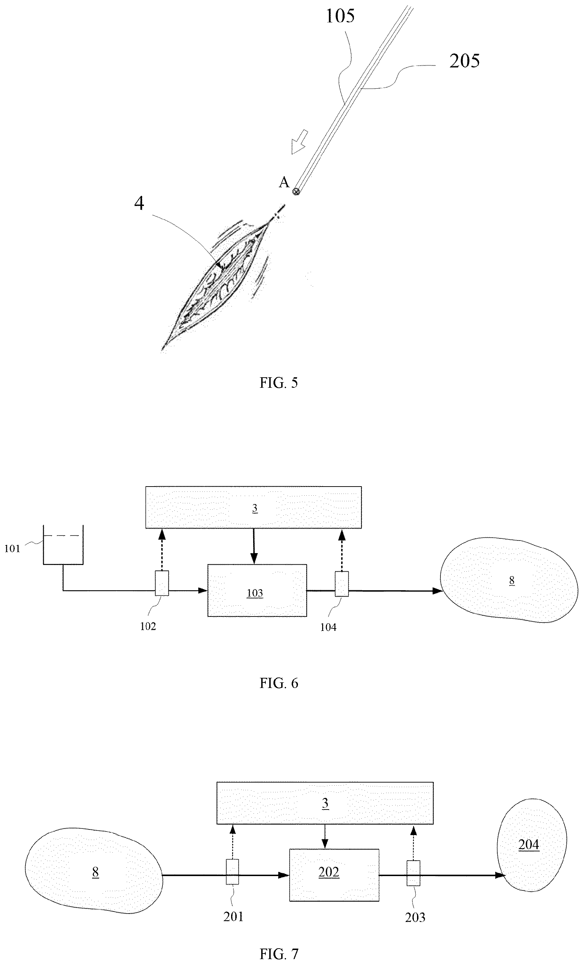

In an exemplary embodiment, please refer to Fig. 6 which is a schematic diagram of an embodiment of an irrigation device in the present application. As shown in the figure, the irrigation device 1 further comprises: an irrigation solution container 101, a first pressure sensor 104 and a first vacuum sensor 102.

Wherein, the irrigation solution container 101 stores an irrigation solution for irrigating the inner cavity 8 of the subcutaneous incision, and communicates with the delivery pump 103 through the first catheter 105. The first catheter 105 is used for delivering the irrigation solution to a preset depth of the inner cavity 8 of the subcutaneous incision, to facilitate healing of the inner cavity 8 of the subcutaneous incision. In one of the ways of present embodiment, the irrigation device 1 can be controlled to intermittently deliver the irrigation solution to the inner cavity 8 of the subcutaneous incision, so that the solidified blood clots, tissue fluid clots and the like in the inner cavity 8 of the subcutaneous incision can be moistened to facilitate removal, and the potential bacterial community reaching a colonization concentration is diluted and cleared along with irrigation solution in drainage, so as to maintain the inner cavity 8 of the subcutaneous incision to be in a cleaning state. In the present embodiment, depending on different patient's conditions, therapeutic regimens and patient's physiques, the intermittency is, for example, a delivery frequency for delivery of drugs in hours or in days. Under a specific implementation state, the irrigation solution container 101 and the delivery pump 103 can also be replaced with a drug delivery device or a drug delivery machine comprising a micro pump or a syringe.

It should be understood that the skin incision may secrete blood, tissue fluid or other body fluids to form effusions during the healing process. If these effusions cannot be timely discharged, they may cause infection, inflammation and even suppuration. In some cases, the effusions that cannot be timely cleared may be clotted in the inner cavity 8 of the subcutaneous incision to form objects difficult to clear, such as blood clots. After being moisturized with the irrigation solution, the blood clots can be dissolved or discharged by means of liquid pressure, so as to facilitate recovery of the subcutaneous incision.

Surgical site infection (SSI for short) is a clinical problem in the world. After the skin is cut/cracked, the deep tissue of the human body will contact with the outside, and may be possibly contaminated by pathogenic bacteria. When the bacterial content in the tissue exceeds 105/g, bacterial colonization will occur, and the bacteria cannot be controlled by the human immune system. With the exponential proliferation of bacteria, tissue necrosis and inflammatory reaction gradually appear in the infected area, and local symptoms of redness, swelling, heat and pain appear; due to an incubation period from bacterial colonization to the appearance of infection symptoms, SSI is usually difficult to be effectively identified and timely treated in the early stage. Typical incision infection usually occurs 7-10 days after the surgery, starting from local redness and swelling, tenderness, and followed by ulceration and pus. Because surgical sutures are easy to be colonized by bacteria, it is very important to remove all the suture knots of subcutaneous suturing as much as possible after SSI, which is also the key to preventing the relapse of SSI. This is because the source of infection of relapsed SSI often comes from residual suture knots.

During the healing process, the irrigation device 1 can deliver the irrigation solution to the inner cavity 8 of the subcutaneous incision through the first catheter 105 and can be used for diluting the bacterial infection that may be or has been generated in the inner cavity 8 of the subcutaneous incision, so that the potential foci of infection reaching a colonization concentration is diluted; meanwhile, the irrigation solution delivered by the irrigation device 1 can also irrigate the infected site in the inner cavity 8 of the subcutaneous incision, and then discharged the irrigation solution in the inner cavity 8 of the subcutaneous incision under the drainage effect of the negative pressure device 2 after irrigation is completed, so as to clear bacteria via drainage, so that the inner cavity 8 of the subcutaneous incision is kept in a cleaning state.

In actual clinical treatment, the amount of the irrigation solution in the irrigation device 1, the time for diluting bacterial infection parts, the irrigation frequency of the irrigation solution, the working frequency of the negative pressure device 2, and the like can be controlled depending on the judged infection situations of the inner cavity 8 of the subcutaneous incision of the patient, etc. For instance, in an exemplary embodiment, the time for irrigating the inner cavity 8 of the subcutaneous incision with the irrigation solution by the irrigation device 1 is for example 2-3 days. Since the irrigation solution container 101 and the delivery pump 103 can also be replaced with the drug delivery device or the drug delivery machine comprising the micro pump or the syringe, when the syringe or other drug delivery devices needing to be manually controlled is used, parameters such as time, frequency and intensity of irrigation can also be manually controlled by an operator.

In an exemplary embodiment, the delivery pump 103 is a diaphragm pump, and the diaphragm pump is a water pump.

It should be understood that the diaphragm pump, also known as a membrane pump and a control pump, is a main type of an actuator. The fluid flow is changed by virtue of dynamic operation by receiving a control signal, the control signal is output by an adjustment control unit. In the process of control, the diaphragm pump is used to receive the control signal of a regulator or a computer, change the flow of the adjusted medium and maintain adjusted parameters to be within a required range, thereby realizing the adjustment and control of parameters during the working process, such as temperature, pressure, flow and liquid level.

In an exemplary embodiment, the first pressure sensor 104 is used for feeding back the sensed fluid pressure value of the first catheter 105 between the delivery pump 103 and the inner cavity 8 of the subcutaneous incision to the control device 3, so as to adjust the rotation speed of the delivery pump 103 to control the output pressure of the irrigation solution. In the process of cleaning the inner cavity 8 of the subcutaneous incision by the irrigation device 1, if the fluid pressure is too high, it will cause secondary injury to the incision and bring pains to the patient. On the contrary, if the fluid pressure is too low, the irrigation effect will be unsatisfactory, and the effusions in the inner cavity 8 of the subcutaneous incision cannot be flushed away, then the effusions still remain in the inner cavity 8 of the subcutaneous incision, which is not conducive to the healing of the incision. In order to avoid the above situations, a first pressure sensor 104 is arranged on the first catheter 105 between the delivery pump 103 and the inner cavity 8 of the subcutaneous incision, the fluid pressure value sensed by the first pressure sensor 104 reflects a fluid pressure value at the output end of the first catheter 105. The fluid pressure value sensed by the first pressure sensor 104 is fed back to the control device 3, so that the control device 3 adjusts the rotation speed of the delivery pump 103, thereby controlling the output pressure of the irrigation solution.

It should be understood that the feedback means that the fluid pressure value sensed by the first pressure sensor 104 is provided to the control device 3, so that the control device 3 adjusts the rotation speed of the delivery pump 103 based on a preset desired pressure value, thereby controlling the output pressure of the irrigation solution. This process is realized by feedback control, namely, a process in which the output information of the system is sent back to the input end and compared with the input information, and their bias is utilized for control. Specifically, the feedback control refers to a fact that actual results are compared after a certain action and task are completed, so as to affect the next action and play a role of controlling. Timely response can be made to the objective effect caused by each step in the implementation process of the plan decision, and accordingly the next implementation plan is adjusted and modified, so that dynamic coordination is reached between the implementation of the plan decision and the original plan itself. The first pressure sensor 104 or the second pressure sensor 203 adopts a pressure sensor with a model comprising but not limited to MPXH6300A.

In an exemplary embodiment, the first vacuum sensor 102 is used for sensing vacuum degree of the first catheter 105 between the delivery pump 103 and the irrigation solution container 101, so as to monitor the exhausting state of the irrigation solution in the irrigation solution container 101. After the irrigation solution in the irrigation solution container 101 is exhausted, it is necessary to timely stop infusion or replenish the irrigation solution, or the air may enter the inner cavity 8 of the subcutaneous incision. Herein, whether the irrigation solution in the irrigation solution container 101 is exhausted can be reflected by arranging a first vacuum sensor 102 on the first catheter 105 between the delivery pump 103 and the irrigation solution container 101 and by detecting the vacuum degree of the first catheter 105 between the delivery pump 103 and the irrigation solution container 101.

In an exemplary embodiment, when the irrigation solution in the irrigation solution container 101 is detected to be exhausted or is about to be exhausted, the detection result is fed back to the control device 3, so that the control device 3 turns off the delivery pump 103, thereby preventing the delivery pump 103 from continuing to work.

It should be understood that the first vacuum sensor 102 is arranged on the first catheter 105 between the delivery pump 103 and the irrigation solution container 101. Therefore, the vacuum degree detected by the first vacuum sensor 102 reflects the vacuum degree of the first catheter 105 between the delivery pump 103 and the irrigation solution container 101. When the first catheter 105 between the delivery pump 103 and the irrigation solution container 101 is filled with liquid or a large amount of liquid flows through, the vacuum degree in the first catheter 105 between the delivery pump 103 and the irrigation solution container 101 is zero or close to zero, but when no liquid or very little liquid flows through the first catheter 105 between the delivery pump 103 and the irrigation solution container 101, it indicates that the irrigation solution in the irrigation solution container 101 has been exhausted or is nearly exhausted while the vacuum degree of the first catheter 105 between the delivery pump 103 and the irrigation solution container 101 will significantly increase. Therefore, after the first vacuum sensor 102 is arranged on the first catheter 105 between the delivery pump 103 and the irrigation solution container 101, whether the irrigation solution in the irrigation solution container 101 is exhausted or nearly exhausted, namely the exhausting state of the irrigation solution in the irrigation solution container 101, is determined through the vacuum degree value detected by the first vacuum sensor 102

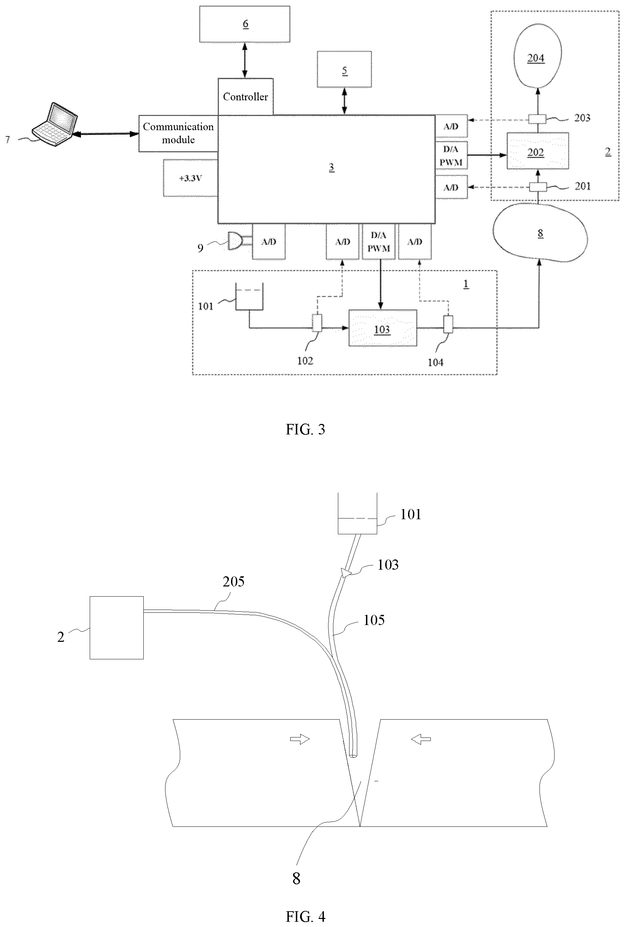

In an exemplary embodiment, please refer to Fig. 3. Fig. 3 is a schematic diagram of an embodiment of an alarming device in a negative pressure drainage and cleaning system for sutureless closed skin incisions of the present application. As shown in the figure, the irrigation device 1 further comprises an alarming device 9 used for outputting an alarming signal when the first vacuum sensor 102 detects that the irrigation solution in the irrigation solution container 101 is exhausted. Herein, when the first vacuum sensor 102 arranged on the first catheter 105 between the delivery pump 103 and the irrigation solution container 101 detects that the irrigation solution in the irrigation solution container 101 is exhausted or is about to be exhausted, an alarm is given by the alarming device 9 to remind replenishment of the irrigation solution or stop working of the irrigation device 1.

It should be understood that the alarming device 9 is a device capable of receiving a control signal from the control device 3 to emit a sound to achieve an alarming function, it comprising but not limited to a buzzer, a voice alarm system, a sound and light alarm, etc.

In an exemplary embodiment, the irrigation device 1 comprises a delivery pump 103, a first catheter 105, an irrigation solution container 101, a first pressure sensor 104 and a first vacuum sensor 102, wherein, the irrigation solution container 101 is internally provided with an irrigation solution for irrigating the inner cavity 8 of the subcutaneous incision. The delivery pump 103 is used for delivering the irrigation solution in the irrigation solution container 101 and controlling the flow, strength and other parameters of the fluid. One end of the first catheter 105 is connected with the irrigation solution container 101, and the other end of the first catheter 105 partially reaches into a preset depth of the inner cavity 8 of the subcutaneous incision, so as to deliver the irrigation solution in the irrigation solution container 101 to the inner cavity 8 of the subcutaneous incision. The first pressure sensor 104 is arranged on the first catheter 105 between the delivery pump 103 and the inner cavity 8 of the subcutaneous incision, thereby feeding back the fluid pressure value to the control device 3, so that the control device 3 adjusts the rotation speed of the delivery pump 103, so as to control the output pressure of the irrigation solution. The first vacuum sensor 102 is arranged on the first catheter 105 between the delivery pump 103 and the irrigation solution container 101, so as to judge whether the irrigation solution in the irrigation solution container 101 is exhausted or is about to be exhausted based on the vacuum degree value detected by the first vacuum sensor 102. When it is detected that the irrigation solution in the irrigation solution container 101 has been exhausted or is about to be exhausted, the alarming device 9 can be triggered by the control device 3 for alarming, or the delivery pump 103 is turned off through the control device 3.

In an exemplary embodiment, the negative pressure device 2 comprises a negative pressure source 202 and a second catheter 205 partially reaching into a preset depth of the inner cavity 8 of the subcutaneous incision, and the negative pressure device 2 is used for generating a negative pressure to force the inner cavity 8 of the subcutaneous incision to be in a closed state during the healing process and draining the effusions in the inner cavity 8 of the subcutaneous incision through the second catheter 205. The second catheter 205 is used for generating the negative pressure to force the inner cavity 8 of the subcutaneous incision to be in a closed state during the healing process, maintaining the transecting tissues to be in a fitting state and timely clearing the oozed blood and oozed fluid in the incision cavity through continuous negative pressure suction. In the actual implementation process, the second catheter 205 of the negative pressure device 2 reaches into the inner cavity 8 of the subcutaneous incision, and then generates the negative pressure to force tissues on both sides of the inner cavity 8 of the subcutaneous incision to be fit with each other in an opposite direction (such as a direction indicated by arrows on both sides of the subcutaneous incision in Fig. 2) to eliminate the inner cavity 8 of the subcutaneous incision, and the closure of the inner cavity 8 of the subcutaneous incision facilitates healing of the incision. It should be understood that in this process, the part of the second catheter 205 in the inner cavity 8 of the subcutaneous incision does not affect the growth of tissues on both sides of the inner cavity 8 of the subcutaneous incision.

It should be understood that during the healing process, the skin incision may secrete blood, tissue fluid or other body fluids to form the effusions. If these effusions are not timely discharged, they will cause infection, inflammation and even suppuration. The effusions in the inner cavity 8 of the subcutaneous incision are drained by the negative pressure so as to avoid unfavorable conditions such as incision infection, inflammation or suppuration, thereby facilitating healing of the incision.

In an exemplary embodiment, please refer to Fig. 7 which is a schematic diagram of an embodiment of a negative pressure device in the present application. As shown in the figure, the negative pressure device 2 further comprises: a collection container 204, a second pressure sensor 203 and a second vacuum sensor 201.

Wherein, the collection container 204 is used for collecting the effusions drained by the second catheter 205 from the inner cavity 8 of the subcutaneous incision. The collection container 204 comprises, but is not limited to, containers that are used for storing liquid, such as a liquid collection bottle and a liquid collection tank. The second catheter 205 collects the effusions drained from the inner cavity 8 of the subcutaneous incision in the collection container 204. When the collection container 204 is full, the collection container 204 can be replaced or the effusions in the collection container 204 can be cleared.

In order to ensure that the irrigation solution container 101 and the collection container 204 are maintained in a sterile working environment, in an exemplary embodiment, the control part of the control device 3 and the irrigation solution container 101 as well as the collection container 204 are integrated in different equipment shells. For example, the control part of the control device 3 (comprising the control device, the delivery pump, and the negative pressure source) is configured in a first shell, the irrigation solution container 101 and the collection container 204 are configured in a second shell, the irrigation solution container 101 and the collection container 204 are physically separated in the second shell, and each container has an independent space that is not communicated with each other, the second shell is provided with catheter interfaces respectively connecting the irrigation solution container 101 and the collection container 204 and communicating with respective catheters, that is, the collection container 204 communicates with the second catheter 205, and the irrigation solution container 101 communicates with the first catheter 105. In the present embodiment, the irrigation solution container 101 and the collection container 204 are detachably connected with the shell, which is convenient for replacement or maintenance.

In another exemplary embodiment, in clinic, for flexible placement, the irrigation solution container 101 and the collection container 204 can also be independently arranged in respective shells, for example, the control part (comprising the control device, the delivery pump, and the negative pressure source) of the control device 3 is configured in the first shell, the irrigation solution container 101 is configured in the second shell, and the collection container 204 is configured in the third shell. In an exemplary embodiment, the second pressure sensor 203 is used for sensing fluid resistance of the second catheter 205 between the negative pressure source 202 and the collection container 204, so as to monitor the filling state of the collection container 204. Please continue to refer to Fig. 1, as shown in the figure, a second pressure sensor 203 is arranged on the second catheter 205 between the negative pressure source 202 and the collection container 204. When the collection container 204 is filled with or is to be filled with the liquid, the fluid resistance in the second catheter 205 between the negative pressure source 202 and the collection container 204 will significantly increase, and the second pressure sensor 203 can detect the fluid resistance in the second catheter 205 between the negative pressure source 202 and the collection container 204 to judge whether the collection container 204 is full or is about to be full, so as to judge the filling state of the collection container 204.

It should be understood that the fluid resistance is usually divided into: the flow of fluid in a pipeline system can be divided into the flow in a uniform straight pipe, resulting in on-way resistance mainly based on surface friction; and the flow in various pipe fittings such as valves, elbows, equipment inlets and outlets, etc., due to the change of flow path direction, change of cross-section area, flow channel bifurcation and convergence, etc., local resistance based on reverse pressure difference or eddy current is generated. When the liquid in the collection container 204 is full, the fluid in the second tube 205 is subject to resistance from the liquid in the collection container 204 and cannot continue to flow into the collection container 204.

In an exemplary embodiment, the negative pressure device 2 further comprises an alarming device 9 for outputting an alarming signal when the second pressure sensor 203 detects that the collection container 204 is filled with or is about to be filled with the effusions. Herein, when the second pressure sensor 203 arranged on the second catheter 205 between the negative pressure source 202 and the collection container 204 detects that the collection container 204 is filled with or is about to be filled with the effusions, an alarm is given through the alarming device 9 to remind the replacement and cleaning of the collection container 204. In other cases, when the second pressure sensor 203 arranged on the second catheter 205 between the negative pressure source 202 and the collection container 204 detects that the collection container 204 is filled with or is about to be filled with the effusions, the second pressure sensor 203 can also feedback the information to the control device 3, so that the control device 3 sends a signal of stopping working to the negative pressure device 2 to turn off the negative pressure device 2.

In an exemplary embodiment, the second vacuum sensor 201 is used for feeding back the sensed vacuum degree of the second catheter 205 between the negative pressure source 202 and the inner cavity 8 of the subcutaneous incision to the control device 3, so as to adjust the power of the negative pressure source 202 to control the generated negative pressure. Please continue to refer to Fig. 1, as shown in the figure, a second vacuum sensor 201 is arranged on the second catheter 205 between the negative pressure source 202 and the inner cavity 8 of the subcutaneous incision. Because the second catheter 205 partially reaches into a preset depth of the inner cavity 8 of the subcutaneous incision, when the negative pressure in the second catheter 205 is too high, it will cause secondary damage to the inner cavity 8 of the subcutaneous incision; when the negative pressure in the second catheter 205 is too low, the inner cavity 8 of the subcutaneous incision cannot be closed and the effusions in the inner cavity 8 of the subcutaneous incision cannot be drained. Therefore, the negative pressure in the second catheter 205 needs to be maintained within an appropriate value range. Meanwhile, when the inner cavity 8 of the subcutaneous incision has already been in a closed state or nearly to be in the closed state, the vacuum degree in the second catheter 205 between the negative pressure source 202 and the inner cavity 8 of the subcutaneous incision is zero or nearly to be zero. Therefore, a second vacuum sensor 201 is arranged on the second catheter 205 between the negative pressure source 202 and the inner cavity 8 of the subcutaneous incision, so that the second vacuum sensor 201 reflects the vacuum degree in the second catheter 205 between the negative pressure source 202 and the inner cavity 8 of the subcutaneous incision, and further reflects whether the negative pressure applied to the inner cavity 8 of the subcutaneous incision is within an ideal range. Further, the second vacuum sensor 201 feeds back the acquired vacuum degree of the second catheter 205 between the negative pressure source 202 and the inner cavity 8 of the subcutaneous incision to the control device 3, the control device 3 adjusts the output power of the negative pressure source 202 according to data fed back by the second vacuum sensor 201, thereby controlling the magnitude of the negative pressure generated by the negative pressure source 202 and avoiding incision damage caused by excessive negative pressure or incomplete removal of effusions caused by insufficient negative pressure.

In an exemplary embodiment, the negative pressure source 202 is a diaphragm pump, and the diaphragm pump is an air pump.

It should be understood that the diaphragm pump, also known as a membrane pump and a control pump, is a main type of an actuator. The fluid flow is changed by virtue of dynamic operation by receiving a control signal, the control signal is output by an adjustment control unit. In the process of control, the diaphragm pump is used to receive the control signal of a regulator or a computer 7, change the flow of the adjusted medium and maintain adjusted parameters to be within a required range, thereby realizing the adjustment and control of parameters during the working process, such as temperature, pressure, flow and liquid level.

In some embodiments, the negative pressure source 202 can be an air storer under negative pressure, or can be a manually or electrically driven device that can reduce the pressure in the sealed volume, such as a vacuum pump, a suction pump, wall suction ports that can be used in many healthcare facilities, or a micro pump, a syringe or a static negative pressure device, or any suitable active or passive suction sources. The negative pressure supply can be contained in other components or can be used in combination with these other components, such as sensors, processing units, alarm indicators, memories, databases, software, display devices, or user interfaces that further facilitate treatment. For example, in some embodiments, the negative pressure source 202 may be combined with other components to form a treatment unit. The negative pressure supply may also have one or more supply ports which are configured to facilitate the linking or coupling of the negative pressure supply to one or more distribution components.

It should be understood that the "negative pressure" disclosed in the present application ordinarily refers to a pressure less than a local ambient pressure, such as an ambient pressure in a local environment outside the sealed treatment environment provided by application of drug. In many cases, the local ambient pressure can also be an atmospheric pressure of a place where the tissue part is located. Alternatively, the pressure can be less than a fluid static pressure associated with the tissues at the tissue part. Unless otherwise indicated, the value of the pressure stated herein is a gage pressure. Similarly, an increase of the mentioned negative pressure typically refers to a reduction in an absolute pressure, while the reduction in the negative pressure typically refers to an increase of the absolute pressure. Although the amount and property of the negative pressure applied to the tissue part can be varied with treatment requirements, however, the pressure is totally in low vacuum, ordinarily called crude vacuum, which is between -5mmHg (-667Pa) and -500 mmHg (-66.7kPa). The common therapeutic range is between -75mmHg (-9.9kPa) and -300mmHg (-39.9kPa).

In an exemplary embodiment, the control device 3 is electrically connected with the irrigation device 1 and the negative pressure device 2, and used for controlling the output pressure or flow of the irrigation device 1 and negative pressure device 2 according to the received feedback, so as to maintain the vacuum degree of the inner cavity 8 of the subcutaneous incision.

It should be understood that the irrigation device 1 outputs the irrigation solution through the delivery pump 103, and the pressure of the output irrigation solution and the flow of the output irrigation solution can be adjusted through the delivery pump 103. When the output pressure is too low, the inner cavity 8 of the subcutaneous incision cannot be effectively irrigated; and when the output pressure is too high, it may cause secondary damage to the subcutaneous incision or increase the pains of the patient. Meanwhile, when the output flow is too small, too little irrigation solution will also affect the cleaning effect of the inner cavity 8 of the subcutaneous incision. When the output flow is too large, the output pressure will be increased to cause secondary damage to the subcutaneous incision or increase the pains of the patient. Therefore, the output pressure and flow of the irrigation device 1 should be controlled within an ideal range, so that no adverse affect will be caused to subcutaneous incision and the patient while ensuring the cleaning effect.

It should be understood that the vacuum degree refers to the degree of thinness of gas under vacuum. If a pressure in the tested equipment is lower than the atmospheric pressure, a vacuum gauge is required for pressure measurement. The value read from the vacuum gauge is called the vacuum degree. The value of the vacuum degree is a numeral value indicating that the actual value of the system pressure is lower than the atmospheric pressure, namely, vacuum degree = atmospheric pressure-absolute pressure, absolute pressure = atmospheric pressure + gauge pressure (-vacuum degree). In the present embodiment, the vacuum degree can indirectly reflect the size of the inner cavity 8 of the subcutaneous incision. For example, when the vacuum degree is high, it reflects a large gap still exists between the two sections of the inner cavity 8 of the subcutaneous incision, indicating that negative pressure is possibly low, and may be insufficient to close the inner cavity 8 of the subcutaneous incision; whereas, when the vacuum degree is zero or nearly to be zero, it reflects that the two sections of the inner cavity 8 of the subcutaneous incision have no gap or have a small gap, indicating that the negative pressure may be in an ideal range or may be too high. When the negative pressure is too high, secondary damage may be caused to the subcutaneous incision or the patient may be suffered from pains. When the negative pressure is kept within an ideal range, it is more conducive to healing of the subcutaneous incision. Therefore, maintaining the vacuum degree of the inner cavity 8 of the subcutaneous incision to be in an ideal range can help closing the inner cavity 8 of the subcutaneous incision without causing secondary damage to the subcutaneous incision or bringing pains to the patient, thereby assisting in better healing of the subcutaneous incision. In the present application, the vacuum degree is mainly detected by the vacuum sensor. The vacuum sensor comprises but is not limited to a vacuum sensor with a model of MPXV6115VC6U.

On the one hand, the control device 3 can control the output pressure and output flow of the irrigation device 1 by sensing the signals fed back by the irrigation device 1, so as to ensure that the output pressure of the irrigation device 1 does not damage the subcutaneous incision and at the same time the effusions and impurities in the inner cavity 8 of the subcutaneous incision are cleared. On the other hand, the negative pressure of the negative pressure device 2 can be controlled by the signal fed back by the negative pressure device 2, so as to ensure that the effusions in the inner cavity 8 of the subcutaneous incision is drained while no damage to the subcutaneous incision, close the inner cavity 8 of the subcutaneous incision, and maintain the vacuum degree of the inner cavity 8 of the subcutaneous incision to be within an ideal range.

In an exemplary embodiment, the negative pressure device 2 can generate a vacuum degree of 70% -80%, such as 70%, 71%, 72%, 73%, 74%, 75%, 76 %, 77%, 78%, 79%, 80%, so as to maintain the vacuum degree of the inner cavity 8 of the subcutaneous incision to be within an ideal range.

In an exemplary embodiment, please continue to refer to Fig. 3, the negative pressure device 2 comprises a collection container 204, a second pressure sensor 203, a negative pressure source 202, a second vacuum sensor 201 and a second catheter 205. Wherein, one end of the second catheter 205 partially reaches into a preset depth of the inner cavity 8 of the subcutaneous incision, and the other end of the second catheter 205 is connected with the collection container 204 through the negative pressure source 202, and the second pressure sensor 203 is arranged on the second catheter 205 between the negative pressure source 202 and the collection container 204 and used for judging whether the collection container 204 is filled or not by detecting the resistance. A second vacuum sensor 201 is arranged on the second catheter 205 between the negative pressure source 202 and the inner cavity 8 of the subcutaneous incision and is used for judging the magnitude of the negative pressure in the inner cavity 8 of the subcutaneous incision by detecting the vacuum degree of the inner cavity 8 of the subcutaneous incision. The control device 3 is respectively connected with the second pressure sensor 203 and the second vacuum sensor 201 through an A/D module, namely a digital-to-analog signal conversion module, and the second pressure sensor 203 feeds back the sensed resistance information to the control device 3, so that the control device 3 adjusts the opening and closing of the negative pressure source 202 or control the alarming device 9 to give an alarm. At the same time, the second vacuum sensor 201 feeds back the sensed vacuum degree information to the control device 3, so that the control device 3 adjusts the rotation speed and flow of the negative pressure source 202 through the duty cycle. The irrigation device 1 comprises an irrigation solution container 101, a first catheter 105, a first vacuum sensor 102, a delivery pump 103 and a first pressure sensor 104. Wherein, one end of the first catheter 105 partially reaches into a preset depth of the inner cavity 8 of the subcutaneous incision, and the other end of the first catheter 105 is connected with the irrigation solution container 101 through the delivery pump 103, a first pressure sensor 104 is arranged on the first catheter 105 between the delivery pump 103 and the inner cavity 8 of the subcutaneous incision and used for detecting the output pressure of the delivery pump 103. The first vacuum sensor 102 is arranged on the first catheter 105 between the delivery pump 103 and the irrigation solution container 101 and used for judging the exhaustion situation of the irrigation solution in the irrigation solution container 101 by detecting the vacuum degree. The control device 3 is respectively connected with the first vacuum sensor 102 and the first pressure sensor 104 through an A/D module, namely a digital-to-analog signal conversion module, and the first vacuum sensor 102 feeds back the sensed vacuum degree to the control device 3, so that the control device 3 controls the alarming of the alarming device 9 or the opening and closing of the delivery pump 103, and the first pressure sensor 104 feeds back the sensed pressure to the control device 3, so that the control device 3 controls the output pressure of the delivery pump 103 through the duty cycle.

In an exemplary embodiment, an incision closing device 4 may be used to help the negative pressure device 2 to close the inner cavity 8 of the subcutaneous incision. The incision closing device 4 can squeeze the edge of the skin, so that the two exposed ends of the skin incision are closely fit, and the inner cavity 8 of the subcutaneous incision is kept in a closed state during the healing and rehabilitation process under the forcing and assistance of the negative pressure device 2, the irrigation device 1 irrigates and delivers the liquid drug to the inner cavity 8 of the subcutaneous incision while sucking the effusions in the inner cavity 8 of the subcutaneous incision, and then the potential foci of infection in the inner cavity 8 of the subcutaneous incision reaching a colonization concentration is diluted and cleaned.

In the embodiments provided by the present application, the incision closing device 4 is used for squeezing the edge of the skin, so that the skin incision is in a closed state during the healing process. Meanwhile, squeezing the edge of the skin can reduce bleeding of the vascular network under the dermis, which facilitates recovery of the incision. The incision closing device 4 is arranged on the peripheral side of the skin incision. In some embodiments, the incision closing device 4 is arranged on the peripheral side of the skin incision by means of adhesion. In some embodiments, the incision closing device 4 comprises at least two closure members 41, and the at least two closure members 41 are respectively arranged on both side edges of the skin incision. In the implementation process, the skin incision is kept in a closed state during the healing process through combination of two closure members 41. In practical applications, the number of closure members 41 can be determined by the length of the incision and the specific form of the closure member 41.

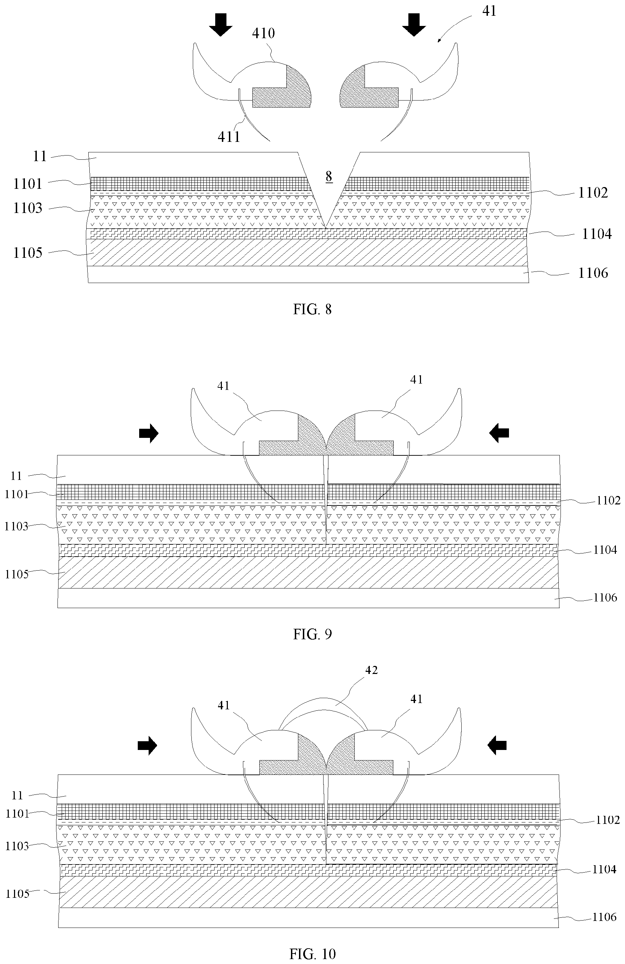

Please refer to Fig. 8, which is a structural schematic diagram of a closure member in an incision closing device in an embodiment of the present application. As shown in the figure, in the embodiment, the incision closing device 4 further comprises a micropore coverage member 40 (not shown in the figure) for covering closure member 41, the micropore coverage member 40 is combined on the closure member 41 according to the shape and structure of the closure member 41 and used to form an entirety together with the closure member 41, so as to facilitate adsorption or suction of secreted fluid oozed from the skin incision and left on the closure member 41 or drug liquid left on the closure member 41. In an exemplary embodiment, the micropore coverage member 40 and the closure member 41 are of an integral structure. In another exemplary embodiment, for example, the micropore coverage member 40 is combined with the closure member 41 by a process such as bonding, so that the micropore coverage member 40 is combined on the closure member 41 according to the shape and structure of the closure member 41.

In some embodiments, the closure member 41 comprises a flexible body 410 which represents an elastic/flexible body material having more than 100% ultimate elongation and significant resilience magnitude. The resilience of the material is a capability of recovering the material from elastic deformation. The elastic/flexible body material for example may comprise but is not limited to natural rubber, polyisoprene, styrene butadiene rubber, chloroprene rubber, polybutadiene, nitrile butadiene rubber, butyl rubber, ethylene propylene rubber, ethylene propylene diene monomer, chlorosulfonated polyethylene, polysulfide rubber, polyurethane, EVA film, copolyester and silicone, etc.

In an exemplary embodiment, the closure member 41 may also be a silicon gel material, a resin material or a silica gel resin material.

In an exemplary embodiment, the material of the microporous coverage member 40 is medical cotton, degreasing cotton, foam, mesh, gauze, sponge, or porous biocompatible materials. The microporous coverage member 40 has air permeability and water adsorption feature, and is used for adsorbing the effusions possibly secreted from the skin incision during the healing process or water of the residual drug that is not absorbed by tissues and left on the skin incision.

In the present embodiment, the closure member 41 comprises a flexible body 410 and a rigid curved needle 411.

The flexible body 410 is adhered to the surface of the skin on the peripheral edge of the skin incision; in the present embodiment, the flexible body 410 is a silica gel material, a resin material or a silica gel resin material. The flexible body 410 is adhered to the surface of the skin on the peripheral edge of the skin incision through an adhesive. In some embodiments, the adhesive is for example a medical adhesive, for example comprising a rapid adhesive mainly based on methyl cyanoacrylate. In the specific implementation process, at least two flexible bodies 410 are respectively adhered to the surface of the skin at two relative sides of the peripheral edge of the skin incision.

The rigid curved needle 411 comprises a root and a curved needle part, wherein the root of the rigid curved needle 411 is buried in the flexible body 410 and firmly arranged on the flexible body 410, and the curved needle part of the rigid curved needle 411 is exposed out of the flexible body 410. In an exemplary embodiment, the root of the rigid curved needle 411 is firmly arranged in the flexible body 410 through a deformed structural design, such as a hook shaped structure or a T-shaped structure.

In an exemplary embodiment, please refer to Fig. 9 which is a schematic diagram of combination of a closure member and an incision in an embodiment of the present application. As shown in the figure, the curved needle part of the rigid curved needle 411 exposed out of the flexible body 410 partially punctures into one side of the skin incision to squeeze the edge of the skin, so that the skin incision is kept in a closed state during the healing process. In the specific implementation process, a doctor first needs to align the skin of the incision (the edges of the skin are aligned, in addition, the edge of the skin is prevented from turning inwards to avoid poor skin healing), and then allows the curved needle part of the rigid curved needle 411 to puncture from one side of the skin incision. Like a state as shown in Fig. 9, the rigid curved needle 411 of the closure member 41 on both sides of the skin incision punctures into the healthy tissue on the side edge of the incision, to squeeze the edge of the skin, so as to reduce the bleeding of the vascular network of the skin dermis, to facilitate healing of the incision and then allow the skin incision to be in a closed state.

In another exemplary embodiment, the curved needle part of the rigid curved needle 411 exposed out of the flexible body 410 partially punctures into the other side of the skin incision, and the other side of the skin incision is hooked using the curved needle part, so that both sides of the skin incision are combined with each other in an opposite direction, and meanwhile the healthy tissues on the opposite sides of the skin incision are driven to be combined with each other in the opposite direction and then the skin incision is closed.

In another exemplary embodiment, the curved needle part of the rigid curved needle 411 exposed out of the flexible body 410 partially punctures into the flexible body 410 arranged on the other side of the skin incision, so that the flexible bodies 410 located on both sides of the skin incision are combined with each other in the opposite direction, and meanwhile the healthy tissues on the opposite sides of the skin incision are driven to be combined with each other in the opposite direction and then the skin incision is closed.

As shown in Fig. 9, a vascular network, a superficial fascia layer, fat, a deep fascia layer, muscles and bones are included in sequence under the tissue of the skin 11. The suturing depth in the present embodiment is in the superficial fascia layer. The problem concerned in the present application is sutureless operation on the superficial fascia part of the skin (i.e., the skin epidermis, dermis, and subcutaneous fat tissue). The puncture depth of the curved needle part of the rigid curved needle 411 exposed out of the flexible body 410 comprising the skin epidermis, dermis and subcutaneous fat tissue of the superficial fascia part of the skin.

In some embodiments, the numbers and lengths of the rigid curved needles 411 arranged on the flexible body 410 can present different implementation states according to different lengths or widths of the appropriate skin incisions. In some embodiments, when a plurality of rigid curved needles 411 are arranged on the flexible body 410, these rigid curved needles 411 are arranged on the elongated flexible body 410 in an equidistantly distributed arrangement.

Please refer to Fig. 10 which is a schematic diagram of another embodiment of a closure member of an incision closing device in the present application. As shown in the figure, in the present embodiment, the closure member 41 also comprises a clamping member 42, and the clamping member 42 is used for clamping the flexible body 410 of the closure member 41 adhered to both sides of the skin incision. The flexible body 410 provides an opposite force to squeeze the edge of the skin under the action of the clamping member 42, to ensure that the skin incision is in a closed state during the healing process. During specific implementation, the clamping member 42 is a medical clip or other parts.

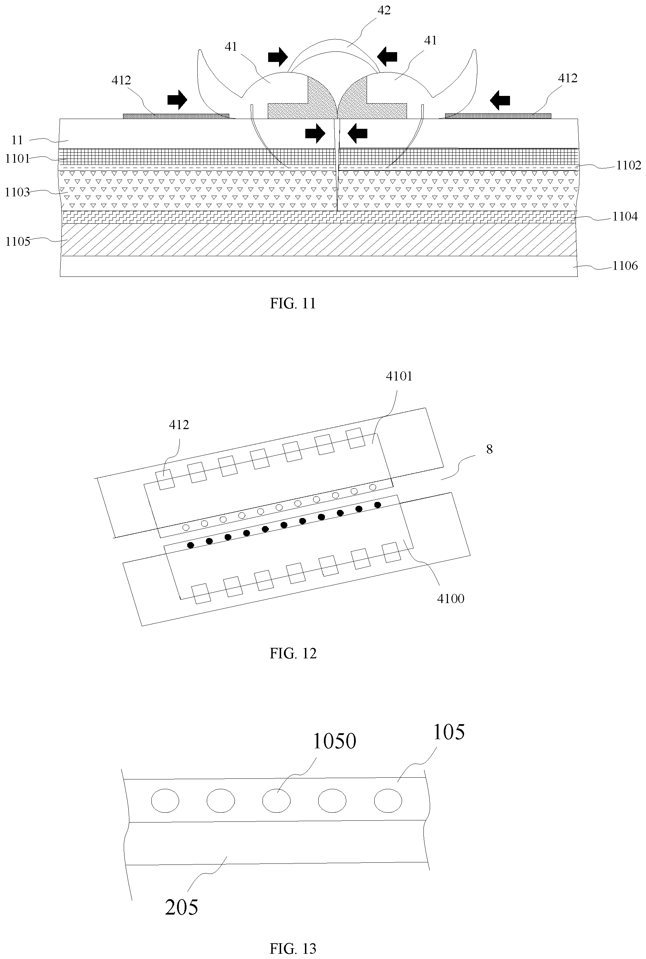

In various embodiments using the closure member 41 comprising the flexible body 410 and the rigid curved needle 411, some auxiliary members 412 can also be used, and the auxiliary members 412 can be used for assisting in adhesion between the closure member 41 and the surface of the skin, and providing a contraction force for closing the skin incision to the closure member 41. Please refer to Fig. 11 which is a schematic diagram of applications of a closure member and an auxiliary member of an incision closing device in an embodiment of the present application.

In another exemplary embodiment, please refer to Fig. 12 which is a structural schematic diagram of a closure member of an incision closing device in another embodiment of the present application. As shown in the figure, in the present embodiment, the closure member 41 comprises: a first flexible body 4100 and a second flexible body 4101.

The first flexible body 4100 is adhered to the surface of the skin at the periphery of the skin incision, the first flexible body 4100 has a first combining part; the second flexible body 4101 is adhered to the surface of the skin at the periphery of the skin incision, the second flexible body 4101 has a second combining part corresponding to the first combining part. The combination between the first combining part and the second combining part provides an opposite force applied to the flexible body 410 on both sides of the skin incision, thereby squeezing the edge of the skin to allow the skin incision to be kept in a closed state during the healing process. In the present embodiment, the first and second flexible bodies 4101 are made of a silica gel material, a resin material or a silicone resin material. The first and second flexible bodies 4101 are adhered to the surface of the skin on two opposite sides of the skin incision by an adhesive. In some embodiments, the adhesive is, for example, a medical adhesive, such as a rapid adhesive mainly based on methyl cyanoacrylate.

In some exemplary embodiments, the first combining part is of a groove structure or a buckle structure, and the second combining part is of a protruding structure that can be limited in the groove structure or a hole or hook structure corresponding to the buckle. For example, a plurality of grooves are formed on the first combining part, and protruding structures corresponding to the plurality of grooves are arranged on the second combining part. The grooves on the first combining part can be replaced with buckle structures. Correspondingly, the protruding structures on the second combining part can be replaced with holes or hook structures corresponding to the buckles; but it is not limited to this. Other combining structures that can realize the mutual combination of the first flexible body 4100 and the second flexible body 4101 can achieve the purpose of the present embodiment, such as the combination of a groove or a hole and a protruding structure, etc.

In some embodiments, please continue to refer to Fig. 12, the incision closing device 4 further comprises an auxiliary member 412, the auxiliary member 412 is used for assisting in adhesion between the closure member 41 and the surface of the skin, and providing a contractile force closing the skin incision to the closure member 41. In an exemplary embodiment, if the skin incision is an incision in a first direction, the auxiliary member 412 is adhered to the periphery of the skin incision, and a force in the second direction which is vertical to the first direction is applied to the closure member 41, the force is called a contraction force in the present embodiment, thereby further helping the closure member 41 to close the skin incision. In the present embodiment, the auxiliary member 412 is adhered to the surface of the skin through its adhesive layer opposite to the surface of the skin. The material of the adhesive layer is, for example, a tape comprising polyurethane coated with an acrylic adhesive.

In the present embodiment, the auxiliary member 412 as a tape can be designed to a ribbon-like structure extending from the center to opposite sides, and the ribbon-like structures extending on both sides can be separated, or integrated. In order to increase the comfort of the patient/sufferer, the ribbon-like structure is suitable for manual extension.

In an exemplary embodiment, the first or second catheter partially reaches into a preset depth of the inner cavity 8 of the subcutaneous incision through a preset position of the skin incision; or the first or second catheter passes through the skin and subcutaneous tissue through a preset position far away from the skin incision to partially reach into a preset depth of the cavity 8 of the subcutaneous incision.

In an exemplary embodiment, please refer to Fig. 4 which is a schematic diagram of an embodiment in which a first or second catheter partially reaches into a preset depth of the inner cavity of the subcutaneous incision from a preset position of a skin incision in the present application. As shown in the figure, the first or second catheter partially reaches into a preset depth of the inner cavity 8 of the subcutaneous incision through the preset position of the skin incision, so as to generate a negative pressure to ensure that the inner cavity 8 of the subcutaneous incision is in a closed state during the healing process, and meanwhile drain the effusions in the inner cavity 8 of the subcutaneous incision, so as to maintain the transecting tissue to be in a fitting state and timely clear the oozed blood and oozed fluid in the incision cavity through continuous negative pressure suction, so that the survival space or environment of bacteria is eliminated as effusions, such as oozed blood and/or oozed fluid in the inner cavity 8 of the subcutaneous incision can be timely cleared. In the present embodiment, the preset position can be any position on the incision, wherein it is more advantageous to the healing of the incision when the preset position is close to the edge of the incision.

In another exemplary embodiment, please continue to refer to Fig. 2, as shown in the figure, the first or second catheter passes through the skin and subcutaneous tissue via a preset position far away from the skin incision to partially reach into a preset depth of the inner cavity 8 of the subcutaneous incision, and accordingly a negative pressure can be generated to ensure that the inner cavity 8 of the subcutaneous incision is in a closed state during the healing process and meanwhile the effusions in the inner cavity 8 of the subcutaneous incision is sucked, so that the survival space or environment is eliminated since the effusions in the inner cavity 8 of the subcutaneous incision can be timely cleared. In the present embodiment, please refer to Fig. 5 which is a schematic diagram of an embodiment in which a first or second catheter passes through skins and subcutaneous tissues via a preset position far away from a skin incision in the present application. The first or second catheter passes through the skin and subcutaneous tissue through the preset position far away from the skin incision (a position as shown at A in Fig. 5) to partially reach into a preset depth of the inner cavity 8 of the subcutaneous incision. The distance refers to a preset position away from the skin incision (a position as shown at A in Fig. 5). In order to facilitate the closure treatment and surface treatment of the skin incision, the first or second catheter reaches into the inner cavity 8 of the subcutaneous incision from additional position rather than through the skin incision, that is, the first or second catheter passes through the inner cavity 8 of the subcutaneous incision from the preset position far away from skin incision. The present embodiment is especially suitable for a long and narrow incision as shown in Fig. 5.

In an exemplary embodiment, the first catheter 105 and the second catheter 205 are integrally formed.

In the present embodiment, please refer to Fig. 13 which is a structural schematic diagram of an embodiment of first and second catheters of the present application. As shown in the figure, the first catheter 105 and the second catheter 205 are integrally formed, and accordingly the quantity of buried catheters can be reduced. The integral formation refers to that one catheter comprises two pipelines that are separated with each other and are not mutually communicated, wherein the first pipeline forms the first catheter 105, and the second pipeline forms the second catheter 205.

In another exemplary embodiment, the second catheter 205 may also be sleeved in the first catheter 105.

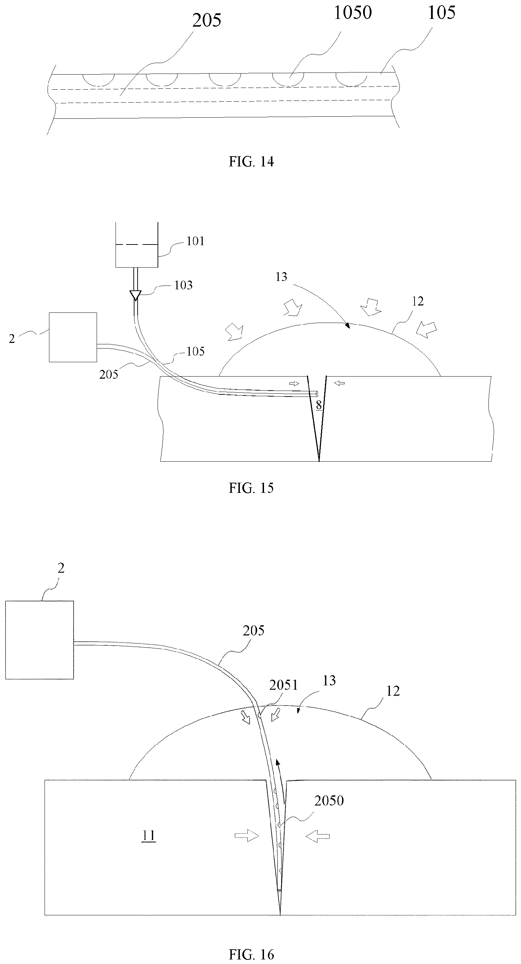

In the present embodiment, please refer to Fig. 14 which is a schematic diagram of another embodiment of first and second catheters of the present application. As shown in the figure, the first catheter 105 and the second catheter 205 are integrally formed, and accordingly the quantity of the buried catheters is reduced. The integral formation refers to that the first catheter 105 and the second catheter 205 are two pipelines that are not mutually communicated, wherein the second catheter 205 is sleeved in the first catheter 105, and the diameter of the first catheter 105 is larger than the diameter of the second catheter 205. Since the thin catheter is sleeved in the thick catheter, the diameter of the whole catheter may not be increased, also the quantity of the catheter is not increased.

In an exemplary embodiment, a plurality of through holes are formed on the wall of the part of the first catheter 105 reaching into a preset depth of the skin incision. Please continue to refer to Fig. 13 to Fig. 14. As shown in the figures, the first catheter 105 is provided with a plurality of through holes 1050, and the first catheter 105 is connected with the irrigation solution container 101, so that the first catheter 105 delivers the irrigation solution in the irrigation solution container 101 to a preset depth of the inner cavity 8 of the subcutaneous incision while the irrigation solution flows to various parts of the subcutaneous incision through the through holes 1050 on the first catheter 105, so as to clean more parts of the subcutaneous incision or deliver drugs. In an exemplary embodiment, the plurality of through holes 1050 are evenly distributed at intervals on the wall of the part of the first catheter 105 reaching into a preset depth of the skin incision, especially for the long and narrow incision, the inner cavity of the incision formed under the skin of the long and narrow incision is usually a long cavity or gap, to ensure that each part of the long cavity or gap can be cleaned by the irrigation solution in the first pipeline or delivered with drugs; in another exemplary embodiment, adapting to different types of skin incisions or different purposes of surgeries, the multiple through holes 1050 on the first catheter 105 can also be designed to be spaced at unequal intervals (that is, the multiple through holes are unevenly distributed at intervals on the wall of the part of the first catheter 105 reaching into a preset depth of the skin incision).

In an exemplary embodiment, the wall of the part of the second catheter 205 reaching into a preset depth in the skin incision is provided with a plurality of through holes, and the second catheter 205 is connected with the negative pressure source 202 so as to generate negative pressure. The plurality of through holes on the second catheter 205 can assist in suction of the gas and liquid in the inner cavity 8 of the subcutaneous incision, thereby improving the suction area and position and further facilitating healing of the subcutaneous incision. In an exemplary embodiment, the plurality of through holes are evenly distributed at intervals on the wall of the part of the second catheter 205 reaching into a preset depth in the skin incision, especially for the long and narrow incision, the inner cavity of the incision formed under the skin of the long and narrow incision is also generally a long cavity or gap, to ensure that the effusions or residual medicine liquid secreted at each of the long cavities or gaps are sucked by the second catheter 205; in another exemplary embodiment, adapting to different types of skin incisions or different purposes of surgeries, multiple through holes on the second catheter 205 may also be designed to be spaced at unequal intervals (that is, the multiple through holes are unevenly distributed at intervals on the wall of the part of the second catheter 205 reaching into a preset depth of the skin incision).

In still another exemplary embodiment, the shape and structure of part of the first catheter 105 and the second catheter 205 that reach into a preset depth of the skin incision may also be designed according to actual needs, for example, according to different desired depths reaching into the skin incision and different tissue structures, the thicknesses or flexibility performances of the first catheter 105 and the second catheter 205 are different, for example, the thicknesses of the same catheter at different positions are different, or the materials of the same catheter at different positions are different, or even the flexibility performances of the same catheter at different positions are different.

In an exemplary embodiment, the first or second catheter is provided with a check valve, to avoid the backflow of the gas or effusions sucked into the catheter which are not beneficial for the healing of the skin incision. In the embodiment, the check valve is for example a duckbill valve or a quadrant valve or a cone-shaped valve, but is not limited thereto. Valve assemblies mechanically or electrically controlled are also applicable to the present embodiment.

It should be understood that the shape of the duckbill valve is similar to that of a duck mouth, so it is called a duckbill valve. The duckbill valve is made of an elastic material, so the outlet of the duck mouth is closed under its own elastic action without a pressure inside. When the pressure inside the duckbill valve gradually increases, the outlet of the duck mouth is enlarged gradually, so that the liquid can be maintained to be discharged at a high flow rate.

It should be understood that the outlet of the quadrant valve is of a quadrant structure. The quadrant valve is made of an elastic material, so the quadrant outlet is closed under its own elastic action without a pressure inside. When the pressure inside the quadrant valve is gradually increased, the quadrant outlet is gradually enlarged, so that the liquid can be maintained to be discharged at a high flow rate.

It should be understood that the sealing surface of the valve core of the cone-shaped valve is a cone-shaped surface. The cone-shaped valve is provided with a cone-shaped valve core at the terminal of a flow passage. All the moving parts are arranged outside the flow passage of the valve, so that the flow passage inside the valve is smooth with large flow and less pressure drop loss. The cone-shaped valve does not produce cavitation and vibration in the whole working range, and still has a good flow control effect when the flow is small.

In an exemplary embodiment, the negative pressure device 2 is also used for maintaining stability of the tissue position at the surface of the skin incision and its peripheral areas by virtue of the generated negative pressure, so as to facilitate recovery of the incision.

In an exemplary embodiment, the negative pressure device 2 comprises: a sealing membrane 12 for adhering to the skin and covering the surface of the skin incision to form a sealed space 13; and a negative pressure channel communicated with the negative pressure source 202, wherein the negative pressure generated from the negative pressure source 202 allows the sealing membrane 12 to provide a contraction force to drive the opposite contraction of the skin incision. In the present embodiment, the negative pressure channel is communicated with the negative pressure source 202 through the second catheter 205, and the negative pressure channel is communicated with the sealed space 13, thereby forming the sealed space 13.

In the present embodiment, the negative pressure generated by the negative pressure device 2 compresses the sealed space 13, so as to maintain the local tissue to be closed towards the position of the incision. At the same time, the negative pressure generated by the negative pressure device 2 also exerts a force on the deep tissue under the incision, so that the potential dead cavity is closed to facilitate healing of the incision. In the present embodiment, the pressure range of the sealed space 13 formed by the sealing membrane 12 adhered to the skin can be set between about 0.001 atmospheric pressure and about 1 atmospheric pressure. In the actual implementation process, the negative pressure value generated by the negative pressure device 2 can be controlled according to the healing degree of the incision, for example, the negative pressure value can be appropriately reduced along with the healing degree of the skin incision, or according to the effusions secreted by the skin incision, for example, when the secreted effusions are increased, the negative pressure value can be appropriately increased to increase the suction strength of the effusions.

In an exemplary embodiment, please refer to Fig. 15 which is a schematic diagram of a sealing and stress structure of a negative pressure device in an embodiment of the present application. As shown in the figure, the sealing membrane 12 is adhered to the skin and covers the surface of the skin incision, thereby forming a sealed space 13. After being pumped by the negative pressure device 2, an inward force is formed in the sealed space 13 inside the sealing membrane 12. The direction of the force is as shown by an arrow in Fig. 15, and the force can be applied to the deep tissue while maintaining the stability of the local tissue position, so as to close the potential dead cavity. In the present embodiment, the sealing membrane 12 bypasses the skin incision to form a sealing area, and makes the sealing area form a sealed space 13. In the present embodiment, the sealing membrane 12 is adhered to the surface of the skin through its adhesive layer relative to the surface of the skin, and the material of the adhesive layer is for example a flexible impermeable material comprising polyurethane coated with an acrylic acid adhesive.

In an exemplary embodiment, the sealing membrane 12 is provided with an observation window made of a light transmitting material. Herein, the sealing membrane 12 may be made of a transparent material. The sealing membrane 12 made of the transparent material can help clinicians to visually observe the healing of the skin incision, so as to timely interfere.

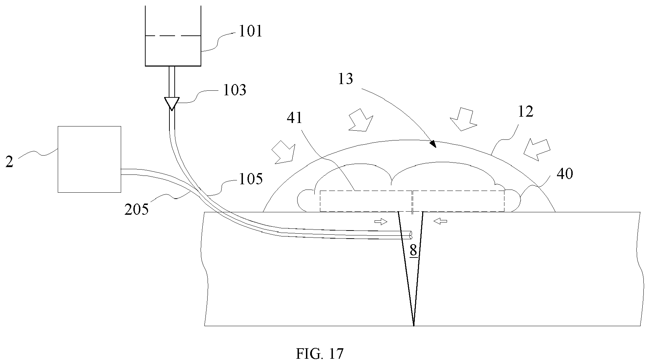

For the specific operating steps of the above embodiment, please refer to Fig. 17 which is a schematic diagram of an application embodiment of the present application. As shown in the figure, the operator embeds the first catheter 105 of the irrigation device 1 and the second catheter 205 of the negative pressure device 2 into the subcutaneous tissue of the skin, and extends the first catheter 105 and the second catheter 205 into the inner cavity 8 of the subcutaneous incision. The negative pressure device 2 can drain the effusions secreted by the inner cavity 8 of the subcutaneous incision through the second catheter 205. At the same time, the inner cavity of the incision is closed/combined inwardly due to the action of a negative pressure, so that the tissues on both sides are oppositely combined. In addition, on the one hand, the irrigation device 1 can also deliver the irrigation solution and the liquid drug to the inner cavity 8 of the subcutaneous incision through the first catheter 105 to facilitate healing; and on the other hand, the skin on both sides outside the incision is closed inwards through the closure member 41, to maintain the skin incision to be in a closed state during the healing process. The compression effect of the closure member 41 on the edge of the skin can reduce bleeding of the vascular network under the skin dermis, and the negative pressure of the negative pressure device 2 can assist in closing the internal cavity 8 of the subcutaneous incision. The operator continues to cover the incision surface with the micropore coverage member 40, to ensure that the secretion on the incision surface can be timely sucked during the healing process. Then, the sealing membrane 12 covers on the outer surface of the micropore coverage member to wrap the whole incision and the microporous coverage member 40 on the incision; on a third hand, the inside of the sealing member 12 is pumped and pressed through the negative pressure channel of the negative pressure device 2, so that tissues in the skin incision and its peripheral area are close to the incision. In addition, the negative pressure generated by the negative pressure device 2 can also exert a certain pressure onto the deep tissue, so that the potential dead cavity is closed, so as to quicken the healing speed of the skin incision, and even a treatment means in which the superficial fascia part of the skin is sutured without needles, and medical tools such as suturing needles and sutures are not used.

In an exemplary embodiment, the negative pressure device 2 comprises a humidity detection component (not shown in the figure) used for providing the detected humidity information in the sealed space 13 to the negative pressure source 202, so as to facilitate the control of the negative pressure output by the negative pressure source 202. The sensor of the humidity detection component is arranged in the sealed space 13 formed by the sealing membrane 12, and the information output port of the humidity detection component is connected with the control device 3 to provide the detected humidity information to the control pressure device, so as to control the negative pressure device 2 to adjust the magnitude of the output negative pressure. In the present embodiment, the humidity detection component is for example a humidity sensor.

In an exemplary embodiment, please refer to Fig. 16 which is a schematic diagram of an embodiment of a second catheter in the present application. The second catheter 205 comprises subcutaneous drainage holes 2050 and negative pressure holes above skin 2051. The subcutaneous drainage holes 2050 are a plurality of through holes arranged on the wall of the part of the second catheter 205 reaching into a preset depth of the skin incision; the negative pressure holes above skin 2051 is communicated with the sealed space 13 to apply a negative pressure to the sealed space 13. The second catheter 205 is connected with a negative pressure source 202, and the negative pressure source 202 provides negative pressure to the second catheter 205. The subcutaneous drainage holes 2050 of the second catheter 205 is located on the wall of the part of the second catheter 205 reaching into a preset depth of the skin incision, the inner cavity 8 of the subcutaneous incision is forced to be in a closed state during the healing process by virtue of the negative pressure generated by the negative pressure source 202, and the effusions in the inner cavity 8 of the subcutaneous incision is drained by the second catheter 205. The negative pressure holes above skin 2051 of the second catheter 205 is located in the sealed space 13 on the skin and used to communicate with the sealed space 13 to apply a negative pressure to the sealed space 13, thereby maintaining the positions of tissue areas in the skin incision surface and its peripheral area to be stable.

In an exemplary embodiment, please continue to refer to Fig. 1. The negative pressure drainage and cleaning system for sutureless closed skin incisions also comprises a computer 7. The computer 7 is connected with the control device 3, and the connection methods comprise but are not limited to communication connection through USB interface or Bluetooth or wireless network connection. The user can set a program on the computer 7 to set the working time, working mode and the like of the irrigation device 1 and the negative pressure device 2. For example, the driving parameters of the irrigation device 1 and the negative pressure device 2 can be set according to different diseases and different treatment methods or according to the characteristics of the actual diseases or incisions.

In an exemplary embodiment, please continue to refer to Fig. 1. The negative pressure drainage and cleaning system for sutureless closed skin incisions also comprises an interaction device 6. The interaction device 6 comprises but is not limited to a touch screen, a display screen, and an operation keyboard, etc. The user can artificially set the control system through the interaction device, so as to control the working state, working time, working mode and the like of the negative pressure device 2 and the irrigation device 1, and the working state, working time, working mode, and the like of the negative pressure device 2 and the irrigation device 1 are known in real time according to information fed back on the display screen.

In an exemplary embodiment, please continue to refer to Fig. 1. The negative pressure drainage and cleaning system for sutureless closed skin incisions also comprises a memory module 5. The memory module 5 comprises but is not limited to memory chips such as SD cards or cloud storage. The memory module 5 can be used for storing the operation information set by the user and operation programs downloaded from a computer 7 as well as data in the actual use process.

To sum up, according to the negative pressure drainage and cleaning system for sutureless closed skin incision of the present application, in a first aspect, under the forcing action of the negative pressure device 2, the inner cavity 8 of the subcutaneous incision is maintained to be in a closed state during the healing and rehabilitation process, the transecting tissues are kept in a fitting state, and the oozed blood and oozed fluid in the incision cavity are timely cleared through continuous negative pressure suction; in a second aspect, the irrigation solution is intermittently delivered to the inner cavity 8 of the subcutaneous incision, so that the solidified blood clots in the inner cavity 8 of the subcutaneous incision are moisturized to facilitate removal, and the potential bacterial community reaching a colonization concentration is diluted and cleared along with the irrigation solution, thereby maintaining the cleaning state in the incision cavity. Meanwhile, the irrigation solution can also be added or replaced with drugs, so as to be delivered to the inner cavity 8 of the subcutaneous incision to help the healing of the subcutaneous incision; in a third aspect, through the feedback control of the negative pressure device 2, the irrigation device 1 and the control device 3, the control device 3 can timely control the magnitude of the negative pressure of the negative pressure device 2 and the irrigation pressure and flow of the irrigation device 1, so as to ensure that the negative pressure of the negative pressure device 2 can play a good role of closure and drainage. The irrigating device 1 has a good irrigating effect and does not have a negative impact on the subcutaneous incision. And in the use process, the control device 3 can trigger the alarming device 9 to alarm based on the feedback information of the vacuum sensor and the pressure sensor, so as to remind the operator to replenish the irrigation solution or replace the collection container 204 in time. The negative pressure generated by the negative pressure device 2 can also maintain the tissue position in the skin incision and its peripheral area to be stable, thus facilitating healing of the tissues. In addition, through the present application, the sutureless closure of the whole tissue above the deep fascia of the skin can be achieved, so as to avoid the transverse scar (commonly known as "centipede foot") on the surface of the skin caused by suture compression/cutting, and no suture knots are left in the superficial fascia, thereby eliminating an important factor causing bacterial colonization and a main inducement leading to relapse of incision infection.

Therefore, the negative pressure drainage and cleaning system for sutureless closed skin incisions of the present application can make the inner cavity 8 of the subcutaneous incision be kept in a closed state during the healing and rehabilitation process without sutures. In such a way, not only the healing rate of the skin incision is quickened, but also a treatment means that the skin is sutured without needles, and no medical tools such as suturing needles and sutures are used, so after the skin incision is healed, there is no process of clearing foreign matters in the incision or incision surface, for example a step and operating process of dismantling or removing the sutures or taking out the suture residues, and then key links and important inducements for bacterial colonization are eliminated, and more importantly, it is ensured that the healed incision does not leave any traces on the surface of the skin, such as "centipede foot", and the surgical incision part is aesthetically pleasing, and further problems in the prior art that traces are easily left after the surgical incision is sutured and the effusions are difficult to discharge are solved. The surgical assistant equipment for sutureless closed skin incisions in the superficial fascia of the skin is especially suitable for the field of beauty and medical treatment.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

## Claims

1. A negative pressure drainage and cleaning system for sutureless closed skin incisions,
comprising:
an irrigation device, comprising a delivery pump and a first catheter partially reaching into a preset depth of inner cavity of a subcutaneous incision, and used for delivering irrigation solution to the preset depth of the inner cavity of the subcutaneous incision through the first catheter;
a negative pressure device, comprising a negative pressure source and a second catheter partially reaching into a preset depth of the inner cavity of the subcutaneous incision, and used for generating a negative pressure to force the inner cavity of the subcutaneous incision to be in a closed state during the healing process and to drain effusions in the inner cavity of the subcutaneous incision through the second catheter; and
a control device, electrically connected with the irrigation device and the negative pressure device, and used for controlling output pressure or flow of the irrigation device according to received feedback and the negative pressure device, so as to maintain vacuum degree of the inner cavity of the subcutaneous incision.

2. The negative pressure drainage and cleaning system for sutureless closed skin incisions according to claim 1, wherein the irrigation device comprises:
an irrigation solution container, storing the irrigation solution for irrigating the inner cavity of the subcutaneous incision, and communicating with the delivery pump through the first catheter;
a first pressure sensor, used for feeding back sensed fluid pressure value of the first catheter between the delivery pump and the inner cavity of the subcutaneous incision to the control device, so as to adjust rotation speed of the delivery pump to control the output pressure of the irrigation solution; and
a first vacuum sensor, used for sensing vacuum degree of the first catheter between the delivery pump and the irrigation solution container, so as to monitor exhausting state of the irrigation solution in the irrigation solution container.

3. The negative pressure drainage and cleaning system for sutureless closed skin incisions according to claim 2, wherein the irrigation device comprises an alarming device used for outputting an alarming signal when the first vacuum sensor monitors that the irrigation solution in the irrigation solution container is exhausted.

4. The negative pressure drainage and cleaning system for sutureless closed skin incisions according to claim 1 or 2, wherein the delivery pump is a diaphragm pump.

5. The negative pressure drainage and cleaning system for sutureless closed skin incisions according to claim 1, wherein the negative pressure device comprises:
a collection container, used for collecting the effusions drained by the second catheter from the inner cavity of the subcutaneous incision;
a second pressure sensor, used for sensing fluid resistance of the second catheter between the negative pressure source and the collection container, so as to monitor filling state of the collection container;
a second vacuum sensor, used for feeding back the sensed vacuum degree of the second catheter between the negative pressure source and the inner cavity of the subcutaneous incision to the control device, so as to adjust power of the negative pressure source to control generated negative pressure.

6. The negative pressure drainage and cleaning system for sutureless closed skin incisions according to claim 5, wherein the negative pressure source is a diaphragm pump.

7. The negative pressure drainage and cleaning system for sutureless closed skin incisions according to claim 1, wherein the preset depth is an interval depth from superficial fascia to a fat layer and a deep fascia layer in tissues of skin.

8. The negative pressure drainage and cleaning system for sutureless closed skin incisions according to claim 1, wherein the first or second catheter partially reaches into the preset depth of the inner cavity of the subcutaneous incision via a preset position of a skin incision; or the first or second catheter passes through skins and subcutaneous tissues via a preset position far away from a skin incision to partially reach into the preset depth of the inner cavity of the subcutaneous incision.

9. The negative pressure drainage and cleaning system for sutureless closed skin incisions according to claim 1, wherein the first catheter and the second catheter are integrated, or the second catheter is sleeved in the first catheter.

10. The negative pressure drainage and cleaning system for sutureless closed skin incisions according to claim 1, wherein the wall of the part of the first or second catheter reaching into the preset depth in the skin incision is provided with a plurality of through holes.

11. The negative pressure drainage and cleaning system for sutureless closed skin incisions according to claim 1, wherein the first or second catheter is provided with a check valve.

12. The negative pressure drainage and cleaning system for sutureless closed skin incisions according to claim 11, wherein the check valve is a duckbill valve or a quadrant valve.

13. The negative pressure drainage and cleaning system for sutureless closed skin incisions according to claim 1, wherein the negative pressure device is also used for maintaining tissue position on surface and peripheral areas of the skin incision to be stable by virtue of the negative pressure that generated.

14. The negative pressure drainage and cleaning system for sutureless closed skin incisions according to claim 13, wherein the negative pressure device comprises:
a sealing membrane, used for adhering to skins and covering surface of the skin incision to form a sealed space; and
a negative pressure channel, communicating the negative pressure source, wherein the sealing membrane provides a shrinkage force to drive opposite shrinkage of the skin incision through the negative pressure generated by the negative pressure source.

15. The negative pressure drainage and cleaning system for sutureless closed skin incisions according to claim 14, wherein the sealing membrane is adhered to the surface of the skin through adhesion layer of the sealing membrane relative to the surface of the skin, and material of the adhesion layer comprises a flexible impermeable material of polyurethane coated with an acrylic adhesive.

16. The negative pressure drainage and cleaning system for sutureless closed skin incisions according to claim 14, wherein the sealing membrane is provided with an observation window made of a light transmitting material.

17. The negative pressure drainage and cleaning system for sutureless closed skin incisions according to claim 14, wherein the negative pressure device comprises a humidity detection component used for providing detected humidity information in the sealed space to the negative pressure source so as to facilitate adjustment of the negative pressure output of the negative pressure source.

18. The negative pressure drainage and cleaning system for sutureless closed skin incisions according to claim 14, wherein the second catheter comprises subcutaneous drainage holes and negative pressure holes above skin, the subcutaneous drainage holes are a plurality of through holes formed on the wall of the part of the second catheter reaching into the preset depth in the skin incision; and the negative pressure holes above skin communicate with the sealed space to apply a negative pressure to the sealed space.
